Europäisches Patentamt

European Patent Office

Office européen des brevets

(19)

(11)  EP 0 718 263 A1

(12)  EUROPÄISCHE PATENTANMELDUNG

(43)  Veröffentlichungstag:
      26.06.1996  Patentblatt 1996/26

(51)  Int. Cl.$^6$: C07C 17/278, C07C 19/16

(21)  Anmeldenummer: 95120171.4

(22)  Anmeldetag: 20.12.1995

(84)  Benannte Vertragsstaaten:
      BE DE FR GB IT

(30)  Priorität: 24.12.1994 DE 4446758

(71)  Anmelder: HOECHST AKTIENGESELLSCHAFT
      D-65926 Frankfurt am Main (DE)

(72)  Erfinder: Werner, Konrad von, Dr.
      D-84518 Garching (DE)

(54)  Metallkatalysierte Herstellung von Perfluoralkyliodid-Telomeren

(57)  Die Selektivität von Kupfer als Katalysator bei der Umsetzung von kurzkettigen Perfluoralkyliodiden mit Tetrafluorethylen wird erhöht, wenn als Cokatalysator ein weiteres Übergangsmetall eingesetzt wird.

## Beschreibung

Mittel- bis längerkettige Perfluoralkyliodide sind Ausgangsmaterialien für die Herstellung von Fluortensiden sowie von hydro- und oleophobierenden Ausrüstungsmaterialien, beispielsweise für Textilien. Andererseits ist insbesondere das n-Perfluoroctyliodid ein Ausgangsmaterial für das n-Perfluoroctylbromid, das im medizinischen Bereich eine erhebliche Bedeutung erlangt hat.

Perfluoralkyliodide werden technisch durch Telomerisation entsprechend der Gleichung

$$Rfl + n\ F_2C{=}CF_2 \longrightarrow Rf(CF_2{-}CF_2)_nI,$$

wobei Rf für Perfluoralkyl mit 1 bis 6 Kohlenstoffatomen und n für eine Zahl von 1 bis etwa 8 steht, hergestellt. Die Reaktion wird entweder durch Erhitzen (beispielsweise US-A 5 268 516) oder durch Radikalbildner (beispielsweise GB-A 1 535 408 oder US-A 5 068 471) gestartet. Bei der thermischen Reaktion bilden sich in erheblichem Ausmaß Perfluoralkane durch Dimerisierung der intermediär gebildeten Perfluoralkylradikale, und bei den radikalischen Reaktionen treten als Nebenprodukte wasserstoffhaltige Verbindungen auf.

Wie sich aus der obengenannten Reaktionsgleichung ergibt, wird die Bildung der im allgemeinen unerwünschten längerkettigen Telomeren durch eine hohe Konzentration des Telogens Rfl zurückgedrängt. Eine Selektivität in Richtung auf erwünschte mittelkettige Telomere wird damit durch geringe Umsätze erkauft, womit ein hoher Destillationsaufwand an zurückzuführendem Telogen und niederkettigen Telomeren verbunden ist.

In einer als "Preliminary Note" gekennzeichneten Publikation beschreiben Chen et al., Journal of Fluorine Chemistry 36 (1987), Seiten 483 bis 489, den Einsatz von Kupfer als Telomerisationskatalysator. Dieses Verfahren hat den Vorteil, daß die Reaktion bereits bei 80 bis 100 °C abläuft und im Verhältnis zu Hochtemperatur-Telomerisationen kürzere Reaktionszeiten erfordert. Bei der Umsetzung von Perfluorethyliodid und Tetrafluorethylen im Molverhältnis von 1 : 2 bis 2 : 1 werden noch relativ große Mengen an unerwünschten längerkettigen Telomeren gebildet.

Überraschenderweise wurde nun gefunden, daß die Selektivität von Kupfer als Katalysator für die Telomerisationsreaktion in Richtung auf die erwünschten mittelkettigen Produkte erhöht werden kann, wenn das Kupfer in Kombination mit einem weiteren Übergangsmetall eingesetzt wird. Die Erfindung betrifft deshalb ein Verfahren zur Herstellung von Perfluoralkyliodid-Telomeren der Formel

$$Rf(CF_2{-}CF_2)_nI,$$

in der Rf für Perfluoralkyl mit 1 bis 6 Kohlenstoffatomen und n für eine Zahl von 1 bis etwa 4 steht, wobei das Maximum der Kohlenstoffatome im erhaltenen Telomerengemisch im Bereich von 6 bis 10 liegt, durch Umsetzung eines Perfluoralkyliodids der Formel Rfl, in der Rf die vorstehend genannte Bedeutung hat, mit Tetrafluorethylen und Kupfer als Katalysator, das dadurch gekennzeichnet ist, daß als Cokatalysator ein weiteres Übergangsmetall eingesetzt wird.

Das genannte weitere Übergangsmetall schließt solche ein, welche die Reaktion für sich allein nicht oder nur schwach katalysieren wie Eisen, Kobalt, Nickel, Chrom, Molybdän, Wolfram oder Titan. Selbstverständlich kann auch mehr als eines dieser schwach- oder nichtaktiven Metalle in Kombination mit dem Kupfer eingesetzt werden. Wenn also im folgenden vereinfacht von dem "anderen" Metall gesprochen wird, so soll damit nicht ausgeschlossen werden, daß neben dem Kupfer mehr als ein schwach- oder nichtaktives Metall Verwendung findet. Es ist auch möglich, neben dem Kupfer auch ein weiteres katalytisch aktives Metall wie Zink, Mangan, Vanadium, Rhenium, Rhodium oder Silber einzusetzen.

Das Kupfer kann in feinverteilter Form in Mischung mit dem anderen Metall eingesetzt werden oder aber als Überzug auf dem anderen Metall. Dieses kann seinerseits auf einem oberflächenreichen Träger fixiert sein. Hierfür kommen die üblichen Katalysatorträger wie Aluminiumoxid, Kieselgur oder Molekularsiebe in Betracht.

Wenn die Reaktion in einem Autoklav erfolgt, dessen Reaktionsraum aus einem der wenig aktiven Metalle wie Stahl oder Nickel besteht beziehungsweise mit diesen Metallen ausgekleidet ist, so können diese Materialien mit einem dünnen Kupferüberzug versehen werden.

Als Telogene werden bevorzugt 2-Iodperfluorpropan, 1-Iodperfluorethan, -butan und -hexan. Es wurde gefunden, daß die Reaktionsgeschwindigkeit beim Einsatz von 1-Iodperfluorbutan und -hexan etwa um den Faktor 1,6 höher liegt als beim 1-Iodperfluorethan. Eine bevorzugte Ausführungsform der Erfindung besteht also darin, diese niederen Telomeren als Telogene - in reiner Form oder in Mischungen - einzusetzen.

Da die erfindungsgemäße Reaktion mehrphasig ist - das Gas Tetrafluorethylen wird mit dem flüssigen Telogen am festen Katalysator umgesetzt - ist für eine gute Durchmischung zu sorgen. Vorteilhaft sind deshalb Trägerkatalysatoren, bei denen die Metallkombinationen auf einen Träger niedrigerer Dichte aufgebracht sind, da so die Flotierbarkeit in der flüssigen Perfluoralkyliodidphase verbessert wird. Weiterhin vorteilhaft ist der Einsatz eines Rührers, durch den das Tetrafluorethylen in die Flüssigphase eingegast wird. Günstig sind auch die sogenannten Strahlreaktoren, bei denen die Flüssigphase zusammen mit dem Katalysator über eine Düse umgepumpt wird. Das Gas - hier Tetrafluorethylen - wird kurz vor der Düse zudosiert. Die in der Düse erzeugten hohen Scherkräfte bewirken einen effektiven Gas-Flüssig-Übergang. Diese Reaktoren eignen sich auch für den kontinuierlichen Betrieb.

Es wurde weiterhin gefunden, daß Wasser - bei Sauerstoffausschluß - nicht zu einer vermehrten Nebenproduktbildung führt und auch die Reaktion nur wenig beeinträchtigt. Es ist also nicht erforderlich, die Ausgangsmaterialien zu trocknen.

Weitere bevorzugte Ausführungsformen der Erfindung ergeben sich aus den folgenden Beispielen.

Beispiele

Die Ergebnisse wurden gaschromatographisch ausgewertet, wobei mittels experimentell kalibrierter Flächenfaktoren eine direkte Auswertung der gefundenen Daten in Form von Gewichtsanteilen erhalten wurde.

Die eingesetzten Perfluoralkyliodide wurden vor Gebrauch mit Kaliumcarbonat-Pulver von Verunreinigungen wie Fluorwasserstoff oder Iod befreit und unter Stickstoff gelagert. Gaschromatographisch ermittelte Reinheit:

| | |
|---|---|
| $F_2C=CF_2$ | 99,98 % |
| $n-C_2F_5I$ | 97,5 % |
| $n-C_4F_9I$ | 99,8 % |
| $n-C_6F_{13}I$ | 98,7 % |
| $i-C_3F_7I$ | 99,5 % |

Beispiele 1 bis 12

Die Umsetzungen wurden in einem 300-ml-Schüttelautoklav aus Edelstahl ($V_4A$, Material 1,4571) durchgeführt. Die angegebene Katalysatormenge wurde unter Stickstoff vorgelegt. Nach einer Druckprobe mit 50 bar Stickstoff wurde der Autoklav bis 1 mbar evakuiert, dann bis -78 °C abgekühlt. Nach Einsaugen der angegebenen Perfluoralkyliodid-Menge wurde bei -78 °C die gewünschte Menge an Tetrafluorethylen (in den Tabellen "TFE") einkondensiert (die Wägung muß rasch erfolgen, um Fehler durch Vereisung des Autoklaven zu vermeiden).

Beispiele 1 bis 3 und Vergleichsbeispiele V1 und V2

69,2 g 1-Iod-n-perfluorbutan wurden mit der angegebenen Katalysatormenge im angegebenen Molverhältnis mit Tetrafluorethylen 5 Stunden bei 100 °C geschüttelt. Die Ergebnisse zeigt die Tabelle 1. In der letzten Spalte ist unter "Σ X" die Summe aller Nicht-RfI-Verbindungen zusammengefaßt. Sie wurden bei der gaschromatographischen Auswertung pauschal mit dem Massefaktor 1,00 gewichtet. Es handelt sich beispielsweise um Tetrafluorethylen, RfH-Verbindungen (die in geringer Menge in den Edukten enthalten sind), Perfluoralkane und Wasser (das beim Dosieren der Proben mit einer gekühlten Spritze eingeschleppt wurde).

In den Beispielen 2 und 3 wurde ein mit 0,25 Gew.-% Kupfer dotiertes Nickelpulver eingesetzt.

Im Beispiel 3 wurde - im Unterschied zu Beispiel 2 - das Tetrafluorethylen in zwei Portionen eindosiert.

Tabelle 1

| Beispiel | Katalysator | | [g] | [mmol] | Molverhältnis $C_4F_9I/TFE$ | Produktverteilung $C_2F_5(CF_2\text{-}CF_2)_n\text{-}I$ [Masse-%], n = 1 | 2 | 3 | 4 | 5 | 6 | 7 | 8 | 9 | 10 | $\Sigma X$ |
|---|---|---|---|---|---|---|---|---|---|---|---|---|---|---|---|---|
| V1 | Cu | | 2,0 | 31,5 | 1,82 | 54,5 | 25,2 | 11,1 | 4,7 | 1,9 | 0,8 | 0,4 | 0,2 | 0,1 | < 0,1 | 1,1 |
| V2 | Ni | | 2,0 | 34,1 | 2,0 | keine Reaktion | | | | | | | | | | |
| 1 | Ni + Cu | | 2,0 | 34,1 | 2,0 | 58,0 | 23,2 | 9,6 | 4,9 | 2,2 | 0,8 | 0,4 | 0,2 | 0,1 | --- | 0,6 |
| | | | 0,05 | 0,8 | | | | | | | | | | | | |
| 2 | Ni + Cu | | 2,0 | 34,1 | 1,82 | 49,6 | 26,0 | 12,9 | 5,9 | 2,7 | 1,1 | 0,5 | 0,2 | 0,1 | < 0,1 | 1,2 |
| | | | 0,05 | 0,8 | | | | | | | | | | | | |
| 3 | Ni + Cu | | 2,0 | 34,1 | 1,82 | 58,6 | 25,2 | 9,4 | 3,3 | 1,1 | 0,4 | 0,2 | 0,1 | --- | --- | 1,8 |
| | | | 0,05 | 0,8 | | | | | | | | | | | | |

Beispiele 4 bis 11

Die Umsetzung erfolgte analog den vorhergehenden Beispielen, jedoch mit den folgenden Katalysatoren: 34 mmol Nickel(II)-acetat und 3,4 mmol Kupfer(II)-acetat wurden in 150 ml entsalztem Wasser gelöst. Die Lösung wurde auf etwa 80 ml konzentriert und mit dem in der Tabelle 2 angegebenen Trägermaterial gerührt. Die Mischung wurde dann bei 15 mbar und 190 °C entwässert und der Katalysator durch Hydrierung bei 180 bar Wasserstoff und 250 °C aktiviert. Hierbei wurde mehrfach entspannt und wieder Wasserstoff aufgepreßt, um Essigsäure und Restwasser zu entfernen. Der aktivierte Katalysator wurde unter Stickstoff aufbewahrt. Er enthält pro Gramm 73,5 mg Nickel und 8,0 mg Kupfer. Für die Beispiele wurden jeweils 5 g Katalysator, also entsprechend 367,5 mg Kupfer (5,78 mmol) und 40,0 mg Nickel (0,68 mmol), eingesetzt. Es wurden jeweils 69,2 g (0,2 mol) 1-Iod-perfluorbutan eingesetzt. Die Ergebnisse zeigt die Tabelle 2.

Tabelle 2

| Beispiel | Träger | Molverhältnis $C_4F_9I$/TFE | Produktverteilung $C_2F_5(CF_2\text{-}CF_2)_n\text{-}I$ [Masse-%], n = | | | | | | | | | |
|---|---|---|---|---|---|---|---|---|---|---|---|---|
| | | | 1 | 2 | 3 | 4 | 5 | 6 | 7 | 8 | 9 | $\Sigma$ X |
| 4 | Zeolith (10 Å) / 1. Einsatz | 2,0 | 48,5 | 30,6 | 12,9 | 4,8 | 1,6 | 0,6 | 0,2 | 0,1 | < 0,1 | 0,6 |
| 5 | Zeolith (10 Å) / 2. Einsatz | 1,82 | 39,3 | 32,7 | 16,1 | 6,9 | 2,7 | 1,1 | 0,5 | 0,2 | 0,1 | 0,4 |
| 6 | Zeolith (10 Å) / 3. Einsatz | 2,0 | 56,2 | 23,7 | 10,8 | 5,1 | 2,2 | 0,9 | 0,4 | 0,2 | 0,1 | 0,4 |
| 7 | Zeolith (10 Å) / 4. Einsatz | 2,0 | 65,1 | 18,8 | 8,2 | 4,1 | 1,9 | 0,8 | 0,4 | 0,2 | 0,1 | 0,4 |
| 8 | Zeolith (3 Å) | 2,0 | 52,6 | 25,1 | 11,2 | 5,6 | 2,8 | 1,2 | 0,5 | 0,2 | 0,1 | 0,7 |
| 9 | $Al_2O_3$ | 2,0 | 57,4 | 22,9 | 9,7 | 5,2 | 2,4 | 1,0 | 0,5 | 0,2 | 0,1 | 0,6 |
| 10 | Kieselgur | 2,0 | 56,0 | 26,3 | 10,4 | 4,0 | 1,5 | 0,5 | 0,2 | 0,1 | --- | 1,0 |
| 11 | --- | 2,0 | 75,0 | 14,2 | 5,0 | 2,1 | 0,9 | 0,5 | 0,3 | 0,2 | 0,1 | 1,6 |

Beispiel 12 und Vergleichsbeispiele V3 und V4

Beispiel 12 wurde analog den Beispielen 1 bis 3 durchgeführt.

Die Vergleichsbeispiele V3 und V4 wurden demgegenüber mit 50 g Perfluorethyliodid ausgeführt. Die Ergebnisse zeigt die Tabelle 3.

Tabelle 3

| Beispiel | Katalysator | | | Molver-hältnis RfI/TFE | Produktverteilung $C_2F_5(CF_2\text{-}CF_2)_n\text{-}I$ [Masse-%], n = | | | | | | | | | |
|---|---|---|---|---|---|---|---|---|---|---|---|---|---|---|
| | | [g] | [mmol] | | 0 | 1 | 2 | 3 | 4 | 5 | 6 | 7 | 8 | $\Sigma$ X |
| V3 | Cu | 2,0 | 31,5 | 2,22 | 63,4 | 11,9 | 7,2 | 5,3 | 3,9 | 2,9 | 1,9 | 1,0 | 0,4 | 2,1 |
| V4 | Fe | 2,0 | 35,8 | 4,0 | 97,2 | 0,1 | | | | | | | | 2,7 |
| 12 | Fe + Cu | 2,0 0,05 | 35,8 0,8 | 2,0 | --- | 58,7 | 24,8 | 9,8 | 4,2 | 1,5 | 0,6 | 0,2 | 0,1 | 0,1 |

Beispiel 13 und Vergleichsbeispiel V4

Analog den vorherigen Beispielen wurden 89,5 g 1-Iod-n-perfluorhexan bei 100 °C umgesetzt. Die Ergebnisse zeigt die Tabelle 4.

Die Unwirksamkeit von Nickel zeigt das Vergleichsbeispiel V2 (Tabelle 1).

Tabelle 4

| Beispiel | Katalysator | | | Molverhält-nis C_6F_{13}I/TFE | Produktverteilung $C_2F_5(CF_2\text{-}CF_2)_n$-I [Masse-%], n = | | | | | | | | |
|---|---|---|---|---|---|---|---|---|---|---|---|---|---|
| | | [g] | [mmol] | | 2 | 3 | 4 | 5 | 6 | 7 | 8 | 9 | $\Sigma X$ |
| V4 | Cu | 2,0 | 31,5 | 2,0 | 53,0 | 24,5 | 9,8 | 3,6 | 1,3 | 0,5 | 0,2 | 0,1 | 6,5 |
| 13 | Ni + Cu | 2,0 | 34,1 | 2,0 | 56,1 | 24,4 | 8,7 | 2,9 | 0,9 | 0,3 | 0,1 | --- | 5,8 |
| | | 0,1 | 1,6 | | | | | | | | | | |

Beispiel 14 und Vergleichsbeispiel V5

Im Beispiel 14 wurden 1200 g, im Vergleichsbeispiel V5 865 g 1-Iod-n-perfluorbutan, jeweils im Molverhältnis 2 : 1 mit Tetrafluorethylen in einem 1-1-Rührkessel bei 90 °C innerhalb von 2,5 Stunden umgesetzt. Der Rührkessel war mit einer Hülse für das Thermoelement und einem Begasungsrührer ausgestattet, wobei das Tetrafluorethylen durch die hohle Rührerwelle zugeführt und über feine Bohrungen am Ende der Rührerblätter in Form feiner Bläschen in die Flüssigphase verwirbelt wurde. Der Begasungsrührer hatte eine maximale Drehzahl von 500 Upm. Die Ergebnisse zeigt die Tabelle 5.

Tabelle 5

| Beispiel | Katalysator | | | $n_1$ [g] | Produktverteilung $C_2F_5(CF_2\text{-}CF_2)_n$-I [Masse-%], n = | | | | | | | |
|---|---|---|---|---|---|---|---|---|---|---|---|---|
| | | [g] | [mmol] | | 1 | 2 | 3 | 4 | 5 | 6 | 7 | $\Sigma X$ |
| V5 | Zn | 15,0 | 229,4 | 865 | 51,1 | 28,5 | 12,5 | 4,4 | 1,3 | 0,3 | 0,1 | 1,8 |
| 14 | Zn + Cu | 15,0 | 229,4 | 1200 | 50,3 | 29,1 | 13,2 | 4,4 | 1,3 | 0,3 | 0,1 | 1,3 |
| | | 0,5 | 7,9 | | | | | | | | | |

Beispiel 15

In dem für die Beispiele 1 bis 12 genannten Autoklav wurden 29,6 g (0,1 mol) Perfluorisopropyliodid mit 5,0 g (0,05 mol) Tetrafluorethylen 5 Stunden bei 100 °C in Gegenwart von 1,0 g (17,0 mmol) Nickel und 0,05 g (0,8 mmol) Kupfer umgesetzt. Die Ergebnisse zeigt die Tabelle 6.

Tabelle 6

| Beispiel | Produktverteilung $(CF_3)_2CF(CF_2\text{-}CF_2)_n$-I [Masse-%], n = | | | | | | | | | | |
|---|---|---|---|---|---|---|---|---|---|---|---|
| | 0 | 1 | 2 | 3 | 4 | 5 | 6 | 7 | 8 | 9 | $\Sigma X$ |
| 15 | 60,5 | 15,3 | 9,0 | 5,9 | 3,3 | 1,8 | 0,9 | 0,5 | 0,2 | 0,1 | 2,5 |

**Patentansprüche**

1.  Verfahren zur Herstellung von Perfluoralkyliodid-Telomeren der Formel

$$Rf(CF_2\text{-}CF_2)_nI \,,$$

in der Rf für Perfluoralkyl mit 1 bis 6 Kohlenstoffatomen und n für eine Zahl von 1 bis etwa 4 steht, wobei das Maximum der Kohlenstoffatome im erhaltenen Telomerengemisch im Bereich von 6 bis 10 liegt, durch Umsetzung eines Perfluoralkyliodids der Formel RfI, in der Rf die vorstehend genannte Bedeutung hat, mit Tetrafluorethylen und Kupfer als Katalysator, dadurch gekennzeichnet, daß als Cokatalysator ein weiteres Übergangsmetall eingesetzt wird.

2. Verfahren nach Anspruch 1, dadurch gekennzeichnet, daß das als Cokatalysator eingesetzte Metall für sich genommen nicht oder nur schwach katalytisch aktiv ist.

3. Verfahren nach Anspruch 2, dadurch gekennzeichnet, daß ein an sich nur schwach oder nichtaktives Metall mit Kupfer überzogen wird.

4. Verfahren nach Anspruch 1, dadurch gekennzeichnet, daß als Cokatalysator ein katalytisch aktives Metall aus der Reihe Zink, Mangan, Vanadium, Rhenium, Rhodium oder Silber eingesetzt wird.

5. Verfahren nach Anspruch 1, 2 oder 4, dadurch gekennzeichnet, daß die Metalle auf einem Träger fixiert sind.

6. Verfahren nach einem oder mehreren der vorgehenden Ansprüche, dadurch gekennzeichnet, daß das Tetrafluorethylen mit einem Rührer in die Flüssigphase eingegast wird.

EP 0 718 263 A1

EUROPÄISCHER RECHERCHENBERICHT

Europäisches
Patentamt

Nummer der Anmeldung

EP 95 12 0171

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 140 254 (HOECHST AG)<br>* das ganze Dokument * | 1 | C07C17/278<br>C07C19/16 |
| A | JOURNAL OF FLUORINE CHEMISTRY,<br>Bd. 36, 1987 LAUSANNE CH,<br>Seiten 483-489,<br>QING-YUN CHEN ET AL. 'Copper-Induced Telomerization of Tetrafluoroethylene with Fluoroalkyl Iodides'<br>* das ganze Dokument * | 1 | |
| | | | RECHERCHIERTE SACHGEBIETE (Int.Cl.6) |
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 2.April 1996 | Bonnevalle, E |